# EUROPEAN PATENT SPECIFICATION

(11) **EP 3 717 457 B1**
(45) Date of publication and mention of the grant of the patent: **21.06.2023**
(21) Application number: 18883082.2
(22) Date of filing: 27.11.2018
(51) Int. Cl.: C07D 233/86, C07C 231/12, C07C 237/30, C07C 333/08

(54) **PROCESS FOR PREPARATION OF ENZALUTAMIDE USING NOVEL INTERMEDIATE**
VERFAHREN ZUR HERSTELLUNG VON ENZALUTAMID UNTER VERWENDUNG EINES NEUEN ZWISCHENPRODUKTES
PROCÉDÉ DE PRÉPARATION D'ENZALUTAMIDE À L'AIDE D'UN NOUVEL INTERMÉDIAIRE

(30) Priority: 28.11.2017 IN 201721042702; 27.07.2018 IN 201821028360
(43) Date of publication of application: 07.10.2020
(73) Proprietor: Aarti Industries Limited, Mulund (W) Mumbai 400080 Maharashtra (IN)
(72) Inventor: DESAI, Parimal Hasmukhlal, Mumbai - 400080 Maharashtra (IN); SEETHARAMAN, Subramanian, District Thane Mumbai- 421204 Maharashtra (IN); NIKAM, Vikas Hiraman, District Thane Mumbai- 421204 Maharashtra (IN); KAMBLE, Kiran Mohan, District Thane Mumbai- 421204 Maharashtra (IN)
(74) Representative: Crease, Devanand John
(86) International application number: PCT/IN2018/050789
(87) International publication number: WO 2019/106691

(56) References cited:
- EP-A2- 2 895 464
- WO-A1-2015/092617
- WO-A1-2016/005875
- WO-A1-2016/005875
- WO-A2-2014/041487
- CN-A- 104 356 068
- CN-A- 104 610 111
- US-A1- 2015 210 649
- US-B2- 9 174 943
- NARESH KUMAR KATARI ET AL: "A novel approach to the synthesis of aryldithiocarbamic acid esters with arylamines and CS 2 in aqueous media", ADVANCES IN APPLIED SCIENCE RESEARCH, vol. 5, no. 3, 2014, pages 349-355, XP055615669,
- EDWARD B. KNOTT: "Heterocyclyl-rhodanines and -2-thiohydantoints", J. Chem. Soc., 1956, pages 1644-1649, XP000943916,
- NARESH KUMAR KATARI et al.: "A novel approach to the synthesis of aryldithiocarbamic acid esters with arylamines and CS 2 in aqueous media", Advances in Applied Science Research, vol. 5, no. 3, 2014, pages 349-355, XP055615669,

## Description

### Field of the invention

The present invention relates to a novel alkyl ester of [4-cyano-3-(trifluoromethyl)phenyl]carbamodithioic acid, more particularly the invention relates to a process for preparation of alkyl ester of [4-cyano-3-(trifluoromethyl)phenyl]carbamodithioic acid and its use in the preparation of Enzalutamide.

### Background of the invention

Testosterone and other male sex hormones, known collectively as androgens, can fuel the growth of prostate cancer cells. Androgens exert their effects on prostate cancer cells by binding to and activating the androgen receptor, which is expressed in prostate cancer cells. When the cancer spreads beyond the prostate and surrounding tissues into distant organs and tissues it is known as metastatic prostate cancer. Metastatic prostate cancer is initially hormone-sensitive and thus responds to hormone therapies. However virtually all hormone sensitive metastatic prostate cancer undergoes changes that convert it to the Castration-resistant state within 18-24 months after initiation of hormonal therapy. The prostate cancer in this state in known as Castration-resistant prostate cancer.

Enzalutamide, chemically known as 4-(3-(4-Cyano-3-(trifluoromethyl)phenyl)-5,5-dimethyl-4-oxo-2-thioxoimidazolidin-1-yl)-2-fluoro-N-methylbenzamide is a non-steroidal androgen receptor antagonist used in the treatment of patients with metastatic castration-resistant prostate cancer. It is also effective in the treatment of certain types of breast cancer. It can be structurally represented as formula (I).

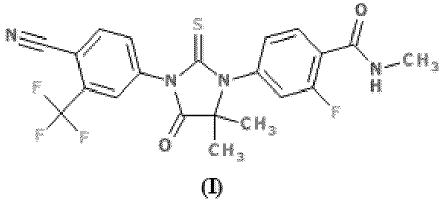

Enzalutamide is first disclosed in the patent US 7,709,517 wherein various diarylhydantoins including diarylthiohydantoins and methods for synthesizing them is disclosed. The process for synthesis of Enzalutamide disclosed in the patent US 7,709,517 is stated in scheme 1 below. The patent also discloses existence of Enzalutamide as a colorless crystal, and it is later designated as Form 1 in one of the PCT application WO2015054804.

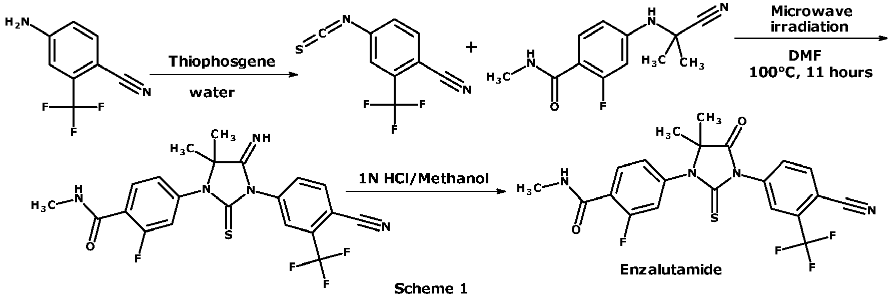

In said process, preparation of isothiocyanate intermediate is an essential step that requires noxious reagent such as thiophosgene, which generates high amount of poisonous phosgene gas. Hence, the use of this intermediate at higher scale production is difficult and requires more labor and extreme care. In addition, said process involves microwave irradiation for cyclization and also yields very low yields hence said process is not feasible at commercial scale, as it requires specialized technology. Moreover, the cited process involves the use of acetone cyanohydrin which is known to be toxic and hazardous and is not recommended for commercial manufacturing. In addition, the cited process involves tedious chromatographic purification which affects the yield and purity of Enzalutamide. This also makes the process not viable for the large scale manufacturing.

Another PCT application WO2016/015423 discloses a process of reacting 4-Amino-2-trifluoromethyl benzonitrile with a source of isothiocyanate compound followed by deprotection yields [4-cyano-3-(trifluoromethyl)phenyl]thiourea.

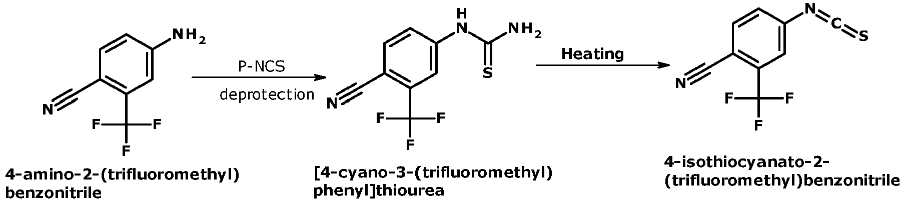

The cited process further involves heating 4-cyano-3-(trifluoromethyl)phenyl]thiourea which results in 4-isothiocyanato-2-(trifluoromethyl)benzonitrile. Accordingly, the cited process avoids use of thiophosgene. However, the examples of said process disclose that the below-mentioned dimer impurities are formed in the process, which need to be removed by purification of 4-isothiocyanato-2-(trifluoromethyl)benzonitrile.

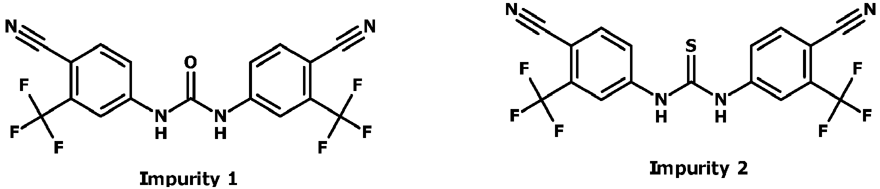

The cited process is incompetent to remove, as said impurities are not removed completely even after purification and are further carried forward in the preparation of Enzalutamide.

One of the patent application CN 104610111 claims preparation of 4-isothiocyanato-2-(trifluoromethyl)benzonitrile by reacting 4-Amino-2-trifluoromethyl benzonitrile with 1,4-diazabicyclo[2.2.2]octane (DABCO) and carbon disulfide to yield DABCO Salt of [4-cyano-3-(trifluoromethyl) phenyl]carbamodithioic acid. The salt is further broken and converted to 4-isothiocyanato-2-(trifluoromethyl) benzonitrile by reacting with bis(trichloromethyl) carbonate (BTC) i.e. triphosgene. The scheme is represented as shown below.

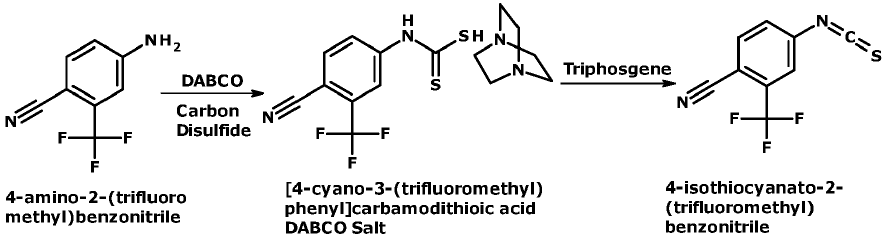

Although, the reaction in said process avoids use of thiophosgene, it involves use of triphosgene which also requires extra precautions during handling. Hence, the reaction of said process requires special technology for handling triphosgene on a large scale production.

One of the patent US 9701641 reports process for preparation of Enzalutamide Form R1, wherein Enzalutamide solution in a single solvent selected from methanol, ethanol, isopropyl alcohol, n-butanol, acetic acid, tetrahydrofuran, toluene, methyl isopropyl ketone, dimethyl sulphoxide, methyl ethyl ketone, acetonitrile, ethyl acetate, xylene, N, N-dimethyl formamide, and N-methyl-2-pyrrolidone; and isolating crystalline Enzalutamide Form 1.

The PCT application WO 2014041487 discloses a process for preparing the amorphous form of Enzalutamide comprising providing a solution of Enzalutamide in a solvent; and isolating amorphous form of Enzalutamide.

WO2016/005875 A1 and US2015/210649 A1 disclose a process for preparing Enzalutamide by using a 2-(trifluoromethyl)-4-isothiocyanato-benzonitrile intermediate compound.

WO2015/092617 A1 discloses a process for preparing Enzalutamide by using an O-phenyl carbamothioate derivative of general formula (IV).

N. K. Katari et al, ADVANCES IN APPLIED SCIENCE RESEARCH, (2014), vol. 5, no. 3, pages 349 - 355, discloses the synthesis of aryldithiocarbamic acid esters with arylamines and CS₂ in aqueous media.

Accordingly, there is a need to provide an improved process for the preparation of Enzalutamide using novel intermediate alkyl ester of [4-cyano-3-(trifluoromethyl)phenyl]carbamodithioic acid, which avoids the use of expensive and hazardous chemicals and reduces impurities in the final product, and further is cost effective and provides better yield and higher purity.

### Objects of the invention

An object of the present invention is to provide a process for preparation of Enzalutamide using novel intermediate giving higher yield and better purity.

Another object of the present invention is to provide a process for Enzalutamide avoiding use of hazardous reagents like triphosgene, thiophosgene and acetone cyanohydrin.

Yet another object of the present invention is to provide a process for preparation of Enzalutamide suitable for industrial production.

Yet another object of the present invention is to provide a process for preparation of crystalline Form 1 of Enzalutamide.

### Summary of the invention

In an aspect, described herein is a process for preparation of Enzalutamide of Formula (I), 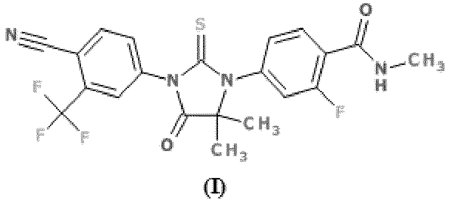 wherein the process for preparation of Enzalutamide of Formula (I), comprises: 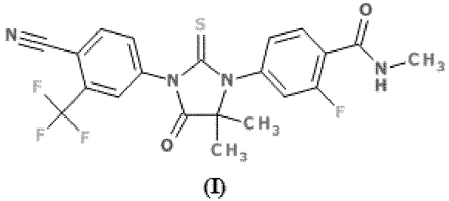 reacting a compound of formula (II) 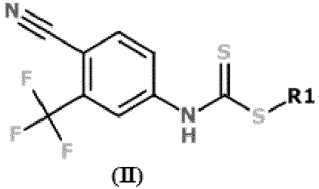
wherein R1 is (1-4C) alkyl, (1-4C) alkyl substituted by ethyl acetate or benzyl acetate, benzyl or benzyl substituted at ortho or para-positions by halo, NO₂ or CN;
with a compound of formula (III) 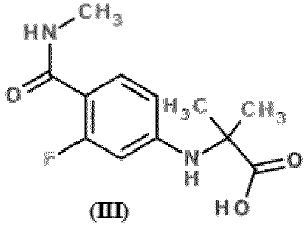
at a temperature of from 60°C to 140°C in presence of a tertiary amine base in a solvent to form Enzalutamide of Formula (I).

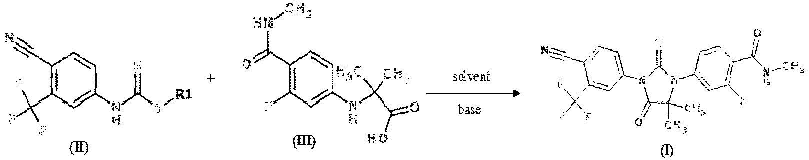

In this embodiment, the compound of formula (II) is alkyl or benzyl ester of [4-cyano-3-(trifluoromethyl)phenyl]carbamodithioic acid wherein R1 is (1-4C) alkyl, (1-4C) alkyl substituted by ethyl acetate or benzyl acetate, benzyl or benzyl substituted at ortho or para-positions by halo, NO2 or CN and the compound of formula (III) is 2-[3-fluoro-4-(methylcarbamoyl)anilino]-2-methyl-propanoic acid. The reaction is carried out at 60°C to 140°C, preferably at 90°C to 130°C and more preferably at 110°C. Further the reaction proceeds in presence of a tertiary amine base in a solvent selected from Toluene, Xylene, cyclohexane, chlorobenzene, dichlorobenzene, and preferably Toluene. The tertiary amine base is selected from di-isopropyl ethylamine (DIPEA), triethylamine, trimethylamine, pyridine, preferably di-isopropylethylamine. Further the predefined tertiary amine base to the solvent ratio is 0.15 (wt/v) to 0.25 (wt/v). In this embodiment, the Enzalutamide of formula (I) has about 93 % to 97 % HPLC purity with about 60 % to 80 % yield.

In another aspect, described herein is a process for preparing the compound of formula (II); 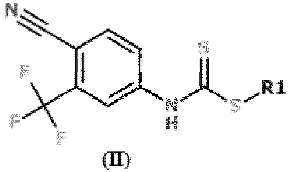 wherein the process of preparation of compound of formula (II) comprises;
1) reacting compound of formula (A) 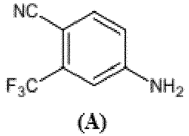 with carbondisulfide at a predefined temperature of from 15°C to 20°C in presence of a amidine base or a 1,4-diazabicyclo[2.2.2]octane (DABCO) or a DABCO derivative and a solvent at room temperature to form a compound of formula (IIA), wherein the solvent used is a ketonic solvent selected from acetone, methyl ethyl ketone, or methyl isobutyl ketone; and 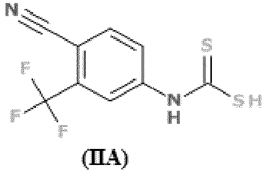
2) reacting the compound of formula (IIA) with a alkylating agent in presence of a solvent at room temperature to form compound of formula (II), wherein the solvent used is a ketonic solvent, a polar aprotic solvent or an ethereal solvent. 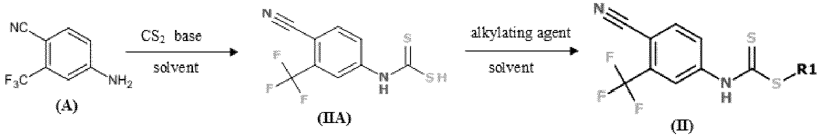
   wherein the compound of formula (II) is an alkyl or benzyl ester of [4-cyano-3-(trifluoromethyl)phenyl]carbamodithioic acid;
   wherein R1 is (1-4C) alkyl, (1-4C) alkyl substituted by ethyl acetate or benzyl acetate, benzyl or benzyl substituted at ortho or para-positions by halo, NO2 or CN.

In this embodiment, the compound of formula (A) is 4-amino-2-(trifluoromethyl)benzonitrile. The reaction proceeds in presence of an amidine base or 1,4-diazabicyclo[2.2.2]octane (DABCO) or DABCO derivative. The amidine base is selected from group of acetamidine, benzamidine, Diazabicyclo[5.4.0]undec-7-ene (DBU), 1,5-Diazabicyclo[4.3.0]non-5-ene (DBN) and imidazolidine etc. The amidine base is preferably 1,8-Diazabicyclo[5.4.0]undec-7-ene (1,8 DBU).

In another embodiment, compound of formula (IIA) is isolated as an amidine base complex or (1,4-diazabicyclo[2.2.2]octane) DABCO salt as compound of formula (IV).

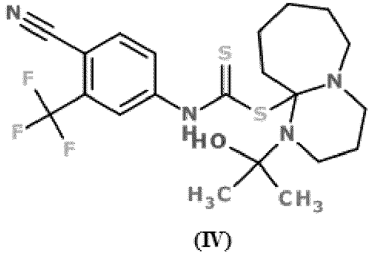

The compound of formula (IV) in is an amidine salt or DABCO salt of [4-cyano-3-(trifluoromethyl)phenyl]carbamodithioic acid. The reaction is preferably carried out in a ketonic solvent selected from acetone, methyl ethyl ketone, methyl isobutyl ketone, preferably acetone. The reaction proceeds at room temperature. Further the amidine base or a 1,4-diazabicyclo[2.2.2]octane (DABCO) or a DABCO derivative to the solvent ratio is 1 to 2.

In another embodiment, the reaction of compound of formula (A) with carbondisulfide (CS₂) is carried out in presence of 1,8-DBU to form 1,8-DBU complex with compound of formula (IIA) of formula (V).

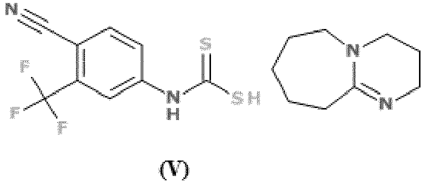

The compound of formula (V) is 1,8-DBU complex of [4-cyano-3-(trifluoromethyl)phenyl]carbamodithioic acid.

In yet another embodiment, the reaction of compound of formula (A) with carbondisulfide is carried out in presence of 1,4-diazabicyclo[2.2.2]octane (DABCO) to form DABCO salt with compound of formula (IIA) of formula (VI).

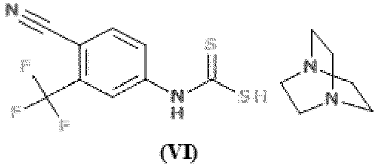

In this embodiment, the compound of formula (VI) is DABCO salt of [4-cyano-3-(trifluoromethyl)phenyl]carbamodithioic acid. Reacting the compound of formula (IIA) with a predefined alkylating agent is done in presence of a predefined solvent at room temperature. The alkylating agent is selected from (1-4 C) alkyl halide, benzyl halide, *o-*nitro benzyl halide, *p*-nitrobenzyl haide, *o*-cyano benzyl halide, *p*-cyano benzyl halide Chloro ethylacetate, bromo ethylacetate, iodo ethylacetate, chloro benzylacetate, bromo benzylacetate and iodo benzylacetate. The reaction is carried out at a room temperature. The solvent is selected from ketonic solvent, polar aprotic solvent or ethereal solvent. In the context of this embodiment, the ketonic solvent is selected from acetone, methyl ethyl ketone or methyl isobutyl ketone. In this embodiment, the Polar aprotic solvent is selected from N-methyl pyrrolidone, Tetrahydrofuran, Ethyl acetate, dimethylformamide, Acetonitrile or DMSO. In this embodiment, the Ethereal solvent is selected from di-tert butyl ether, dibutyl ether, diethyl ether, diisopropyl ether, dimethoxy ethane, dimethoxymethane, 1,4-dioxane, methoxyethane, Methyl tert-butyl ether, THF and 2-methyl THF. The alkylating agent to the solvent ratio is 0.15 to 0.3. In this embodiment, the compound of formula (II) has about 91 % to 98 % HPLC purity with about 50 % to 70 % yield.

Also described herein is a process for preparation of a compound of formula (III); wherein the process for preparation of compound of formula (III) comprises: 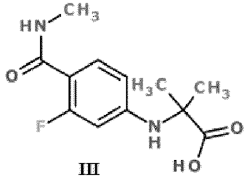
i). reacting a compound of formula (B) 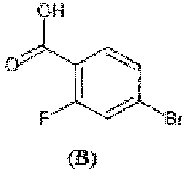 with a predefined chlorinating agent in presence of a mixture of a predefined ketonic solvent and a predefined polar aprotic solvent at a predefined temperature to form compound of formula (C); 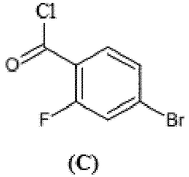
ii). reacting compound of formula (C) with compound of formula (1) in presence of a predefined solvent at room temperature to form compound of formula (D); and 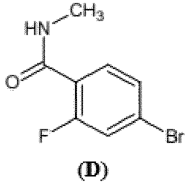
iii). reacting compound of formula (D) with compound of formula (2) in presence of a predefined solvent, a predefined catalyst, a predefined base and a predefined ligand at a predefined temperature to form compound of formula (III).

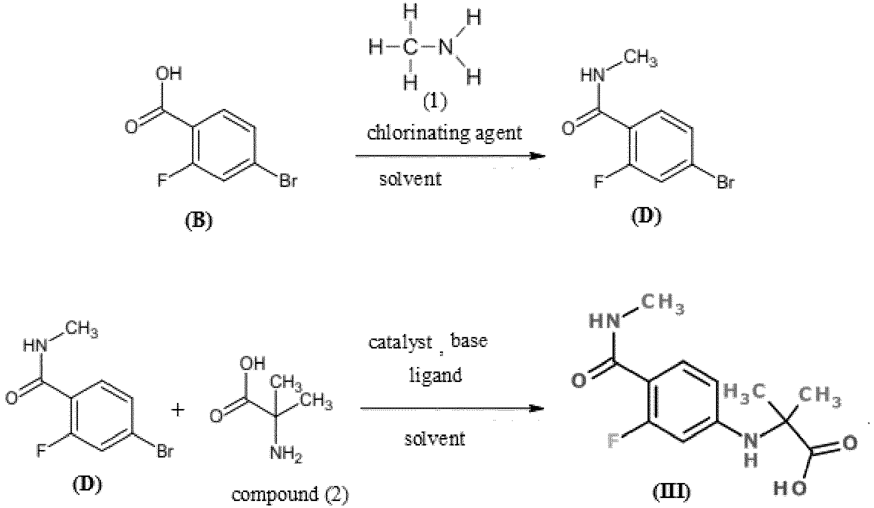

In this example, compound of formula (B) is 4-bromo-2-fluorobenzoic acid. The compound of formula (C) is 4-bromo-2-fluorobenzoyl chloride. The chlorinating agent is thionyl chloride. The reaction is preferably carried out in ketonic solvent selected from toluene and in presence of a polar aprotic selected from dimethylformamide at predefined temperature ranging from 60°C to 85°C, preferably at 70°C to 75°C. The compound of formula (C) is taken as is in the next reaction without isolation. The compound of formula (1) is monomethylamine and compound of formula (D) is 4-bromo-2-fluoro-N-methylbenzamide. Further 40% solution of monomethylamine is preferably used. The reaction is carried out at room temperature in Toluene. The compound of formula (2) is 2-amino isobutyric acid and is reacted with compound of formula (D). The reaction is carried out in a solvent and a catalyst, a base and a ligand. The solvent is selected from dimethyl formamide, dimethylacetamide, DMSO, Acetonitrile, N-methylpyrrolidone. The catalyst is selected cuprous halide selected from cuprous chloride, cuprous bromide, cuprous iodide, preferably cuprous chloride. The base used is alkali metal carbonate selected from sodium carbonate, potassium carbonate and calcium carbonate. The ligand is selected from N,N-dimethylaniline and its derivatives, 3-(dimethylamino)cyclohex-2-en-1-one, N,N-dimethyl-4-(phenylmethyl)benzenamine, 2-(4-N,N-dimethylaminophenyl)naphthalene, N,N-dimethylamino-4-cyclohexylbenzene, 1-(4-N,N-dimethylaminophenyl)naphthalene, 2-(N,N-dimethylamino)-1,4-benzoquinone. N,N-dimethylaniline derivative is selected from O-*halo*-N,N-dimethylaniline, m-*halo*-N,N-dimethylaniline, p-*halo*-N,N-dimethylaniline, *m*-Trifluoromethyl-N,N-Dimethylaniline, p-Trifluoromethyl-N,N-Dimethylaniline, o-Trifluoromethyl-N,N- Dimethylaniline, 4-benzhydryl-N,N-dimethylaniline, wherein halo is selected from Cl, Br, I and F.

Also described herein is a process for purification of a Enzalutamide of formula (I) wherein the process for a preparation of pure Enzalutamide of formula (I) comprising purification process comprises:
I) dissolving crude Enzalutamide of formula (I) in a predefined alcoholic solvent and heating to a predefined temperature to form a clear solution; and
II) cooling the solution at a predefined temperature to isolate pure Enzalutamide. In this example, the alcoholic solvent is selected from methanol, ethanol, isopropanol, butanol. Heating is done at a predefined temperature selected from 55°C to 65°C and the solution is cooled at a predefined temperature selected from 0°C to 10°C. The pure Enzalutamide has about 99.7% to 99.95% HPLC purity with about 50 % to 60 % yield.

The process of the present invention may further comprise the steps of:
a) preparing a solution of Enzalutamide in a mixture of solvent selected from methylene dichloride (MDC), acetone, toluene and methanol; optionally wherein the ratio of Toluene to MDC is 1 to 3 v/v, the ratio of Acetone to MDC is 50 to 70 v/v; and the ratio of MDC to Methanol is 11 to 15 v/v; and
b) isolating the crystalline Form 1 of Enzalutamide by either:
   i. adding the solution formed in step a) to a mixture of chilled or a pre-cooled solvent, optionally n-heptane at -25 to -30 °C; or
   ii. by evaporating the solvent without vacuum and applying a predefined vacuum; optionally wherein the predefined vacuum is in the range of 600-750 mm Hg.

The predefined volume/volume percent of methanol used in step a) ranges from 0.1 v/v to 0.3 v/v, preferably 0.3 v/v.

Further described herein is process for preparation of amorphous form of Enzalutamide, wherein the process comprises:
A) preparing solution of pure Enzalutamide in a predefined volume of a predefined mixture of solvent; and
B) isolating amorphous form of Enzalutamide.

In this example, the predefined solvents in step A) are selected from methylene dichloride and methanol. The ratio of the solvent mixture is the ratio of the solvent is 7 to 9 v/v. The predefined volume/volume percent of methanol used in step A) is 0.5 v/v to 2 v/v. The methylene dichloride layer in step B) is evaporated at temperature ranging from 50°C to 55°C. Sudden precipitation of Amorphous Form of Enzalutamide is observed to yield Amorphous Form of Enzalutamide having purity of 99.7% to 99.95% HPLC purity with yield of 90 % to 99 % yield (on wt/wt basis).

### Brief description of the drawings

Fig 1 is XRD pattern of crystalline Form 1 of Enzalutamide obtained by process of the present invention.
Fig 2 is XRD pattern of amorphous form of Enzalutamide obtained by process described herein.

### Detailed description of the invention

The foregoing objects of the present invention are accomplished and the problems and shortcomings associated with the prior art, techniques and approaches are overcome by the present invention as described below in the preferred embodiments.

All materials used herein were commercially purchased as described herein or prepared from commercially purchased materials as described herein.

Although specific terms are used in the following description for sake of clarity, these terms are intended to refer only to particular structure of the invention selected for illustration in the drawings and are not intended to define or limit the scope of the invention.

References in the specification to "preferred embodiment" means that a particular feature, structure, characteristic, or function described in detail thereby omitting known constructions and functions for clear description of the present invention.

In a preferred embodiment, a process for preparation of Enzalutamide of Formula (I) is disclosed. The process comprises: 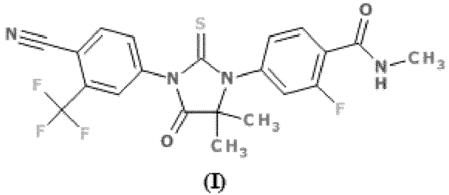 reacting a compound of formula (II) 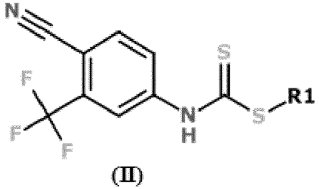
wherein R1 is (1-4C) alkyl, (1-4C) alkyl substituted by ethyl acetate or benzyl acetate, benzyl or benzyl substituted at ortho or para-positions by halo, NO₂ or CN;
with a compound of formula (III) 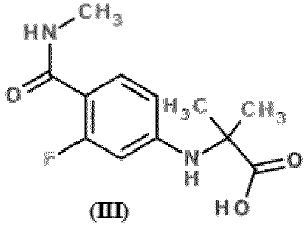
at a predefined temperature in presence of a predefined tertiary amine base in a predefined solvent to form Enzalutamide of Formula (I).

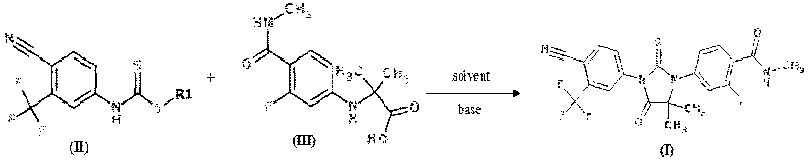

In this embodiment, the compound of formula (II) is alkyl or benzyl ester of [4-cyano-3-(trifluoromethyl)phenyl]carbamodithioic acid wherein R1 is (1-4C) alkyl, (1-4C) alkyl substituted by ethyl acetate or benzyl acetate, benzyl or benzyl substituted at ortho or para-positions by halo, NO2 or CN and the compound of formula (III) is 2-[3-fluoro-4-(methylcarbamoyl)anilino]-2-methyl-propanoic acid. The reaction is carried out at 60°C to 140°C, preferably at 90°C to 130°C and more preferably at 110°C. Further the reaction proceeds in presence of a tertiary amine base in a solvent selected from Toluene, Xylene, cyclohexane, chlorobenzene, dichlorobenzene, and preferably Toluene.

The tertiary amine base is selected from di-isopropyl ethylamine (DIPEA), triethylamine, trimethylamine, pyridine, preferably di-isopropylethylamine. Further the predefined tertiary amine base to the solvent ratio is 0.15 to 0.25 (wt/v).

In this embodiment, the Enzalutamide of formula (I) has about 93 % to 97 % HPLC purity with about 60 % to 80 % yield.

In another embodiment, described herein is a process for preparing the compound of formula (II); 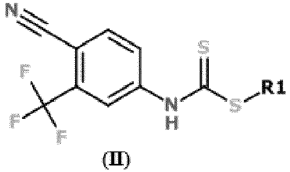 wherein the process of preparation of compound of formula (II) comprises;
1) reacting compound of formula (A) 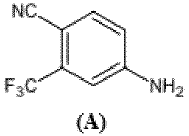 derivative and a predefined solvent at room temperature to form a compound of formula (IIA); and 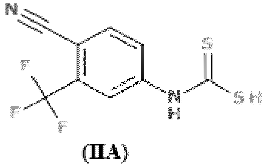
2) reacting the compound of formula (IIA) with a predefined alkylating agent in presence of a predefined solvent at room temperature to form compound of formula (II). 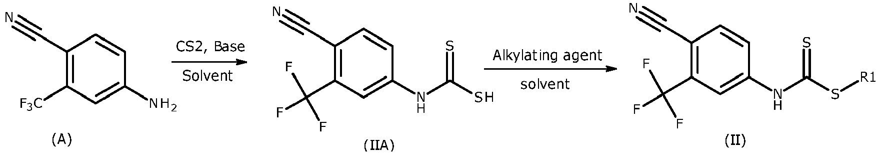
   wherein the compound of formula (II) is an alkyl or benzyl ester of [4-cyano-3-(trifluoromethyl)phenyl]carbamodithioic acid;
   wherein R1 is (1-4C) alkyl, (1-4C) alkyl substituted by ethyl acetate or benzyl acetate, benzyl or benzyl substituted at ortho or para-positions by halo, NO2 or CN.

In this embodiment, the compound of formula (A) is 4-amino-2-(trifluoromethyl)benzonitrile. The reaction proceeds in presence of an amidine base or 1,4-diazabicyclo[2.2.2]octane (DABCO) or DABCO derivative. The amidine base is selected In this embodiment, the compound of formula (A) is 4-amino-2-(trifluoromethyl)benzonitrile. The reaction proceeds in presence of an amidine base or 1,4-diazabicyclo[2.2.2]octane (DABCO) or DABCO derivative. The amidine base is selected from group of acetamidine, benzamidine, Diazabicyclo[5.4.0]undec-7-ene (DBU), 1,5-Diazabicyclo[4.3.0]non-5-ene (DBN) and imidazolidine etc. The amidine base is preferably 1,8-Diazabicyclo[5.4.0]undec-7-ene (1,8 DBU).

In another embodiment, compound of formula (IIA) is isolated as an amidine base complex or (1,4-diazabicyclo[2.2.2]octane) DABCO salt as compound of formula (IV).

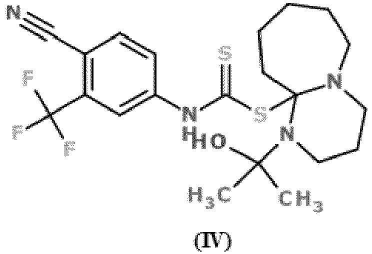

The compound of formula (IV) in is an amidine salt or DABCO salt of [4-cyano-3-(trifluoromethyl)phenyl]carbamodithioic acid.

The reaction is preferably carried out in a ketonic solvent selected from acetone, methyl ethyl ketone, methyl isobutyl ketone, preferably acetone. The reaction proceeds at room temperature.

In another embodiment, the reaction of compound of formula (A) with carbondisulfide is carried out in presence of 1,8-DBU to form 1,8-DBU complex with compound of formula (IIA) of formula (V).

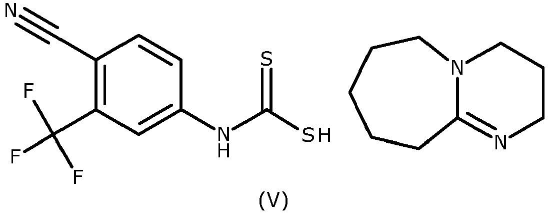

The compound of formula (V) is 1,8-DBU complex of [4-cyano-3-(trifluoromethyl)phenyl]carbamodithioic acid.

In yet another embodiment, the reaction of compound of formula (A) with carbondisulfide is carried out in presence of 1,4-diazabicyclo[2.2.2]octane (DABCO) to form DABCO salt with compound of formula (IIA) of formula (VI).

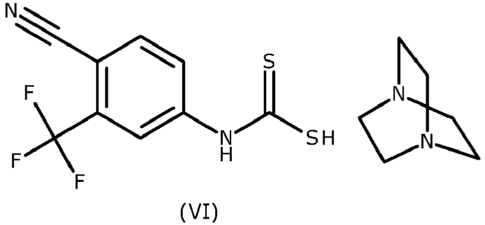

The compound of formula (VI) is DABCO salt of [4-cyano-3-(trifluoromethyl)phenyl]carbamodithioic acid.

The compound of formula (VI) is DABCO salt of [4-cyano-3-(trifluoromethyl)phenyl]carbamodithioic acid.

In this embodiment, wherein reacting the compound of formula (IIA) with a predefined alkylating agent is done in presence of a predefined solvent at room temperature. The alkylating agent is selected from (1-4 C) alkyl halide, benzyl halide, *o*-nitro benzyl halide, *p*-nitrobenzyl haide, *o*-cyano benzyl halide, *p*-cyano benzyl halide Chloro ethylacetate, bromo ethylacetate, iodo ethylacetate, chloro benzylacetate, bromo benzylacetate and iodo benzylacetate. In this embodiment, the reaction is carried out at a room temperature. In the context of this embodiment, the solvent is selected from ketonic solvent, polar aprotic solvent or ethereal solvent. In the context of this embodiment, the ketonic solvent is selected from acetone, methyl ethyl ketone or methyl isobutyl ketone. In this embodiment, the Polar aprotic solvent is selected from N-methyl pyrrolidone, Tetrahydrofuran, Ethyl acetate, dimethylformamide, Acetonitrile or DMSO. In this embodiment, the Ethereal solvent is selected from di-tert butyl ether, dibutyl ether, diethyl ether, diisopropyl ether, dimethoxy ethane, dimethoxymethane, 1,4-dioxane, methoxyethane, Methyl tert-butyl ether, THF and 2-methyl THF.

Further in this embodiment, the alkylating agent to the solvent ratio is 0.15 to 0.3.

In this embodiment, the compound of formula (II) has about 91 % to 98 % HPLC purity with about 50 % to 70 % yield..

In another embodiment, a process for preparation of a compound of formula (III) is disclosed.
wherein the process for preparation of compound of formula (III) comprises: 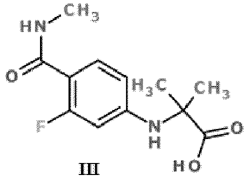
i). reacting a compound of formula (B) 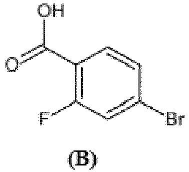 with a predefined chlorinating agent in presence of a mixture of a predefined ketonic solvent and a predefined polar aprotic solvent at a predefined temperature to form compound of formula (C); 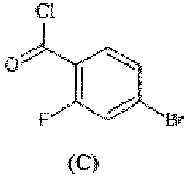
ii). reacting compound of formula (C) with compound of formula (1) in presence of a predefined solvent at room temperature to form compound of formula (D); and 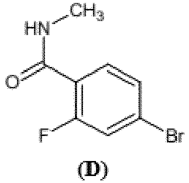
iii). reacting compound of formula (D) with compound of formula (2) in presence of a predefined solvent, a predefined catalyst, a predefined base and a predefined ligand at a predefined temperature to form compound of formula (III).

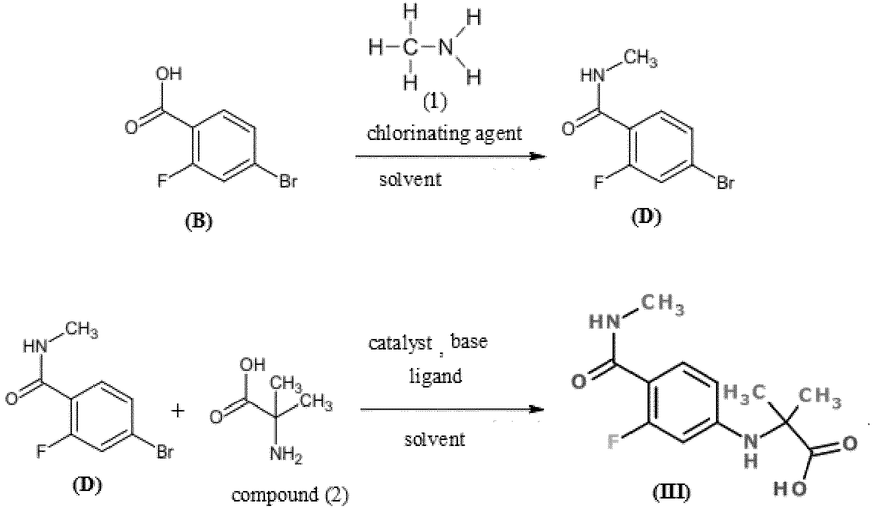

In this embodiment, compound of formula (B) is 4-bromo-2-fluorobenzoic acid. The compound of formula (C) is 4-bromo-2-fluorobenzoyl chloride. The chlorinating agent is thionyl chloride. The reaction is preferably carried out in ketonic solvent selected from toluene and in presence of a polar aprotic selected from dimethylformamide at predefined temperature ranging from 60°C to 85°C, preferably at 70°C to 75°C.

In this embodiment, the compound of formula (C) is taken as is in the next reaction without isolation. The compound of formula (1) is monomethylamine and compound of formula (D) is 4-bromo-2-fluoro-N-methylbenzamide. Further 40% solution of monomethylamine is preferably used. The reaction is carried out at room temperature in Toluene.

In this embodiment, the compound of formula (2) is 2-amino isobutyric acid and is reacted with compound of formula (D). The reaction is carried out in a solvent and a catalyst, a base and a ligand. The solvent is selected from dimethyl formamide, dimethylacetamide, DMSO, Acetonitrile, N-methylpyrrolidone. The catalyst is selected cuprous halide selected from cuprous chloride, cuprous bromide, cuprous iodide, preferably cuprous chloride. The base used is alkali metal carbonate selected from sodium carbonate, potassium carbonate and calcium carbonate. The ligand is selected from N,N-dimethylaniline and its derivatives, 3-(dimethylamino)cyclohex-2-en-1-one, N,N-dimethyl-4-(phenylmethyl)benzenamine, 2-(4-N,N-dimethylaminophenyl)naphthalene, N,N-dimethylamino-4-cyclohexylbenzene, 1-(4-N,N-dimethylaminophenyl)naphthalene, 2-(N,N-dimethylamino)-1,4-benzoquinone. N,N-dimethylaniline derivative is selected from O-*halo*-N,N-dimethylaniline, m*-halo-*N,N-dimethylaniline, p-*halo-*N,N-dimethylaniline, *m*-Trifluoromethyl-N,N-Dimethylaniline, p-Trifluoromethyl-N,N-Dimethylaniline, o-Trifluoromethyl-N,N- Dimethylaniline, 4-benzhydryl-N,N-dimethylaniline, wherein halo is selected from Cl, Br, I and F.

In another embodiment, a process for purification of Enzalutamide of formula (I) is disclosed, wherein the process for a preparation of pure Enzalutamide of formula (I) comprising purification process comprises:
I) dissolving crude Enzalutamide of formula (I) in a predefined alcoholic solvent and heating to a predefined temperature to form a clear solution; and
II) cooling the solution at a predefined temperature to isolate pure Enzalutamide.

In this embodiment, the alcoholic solvent is selected from methanol, ethanol, isopropanol, butanol. Heating is done at a predefined temperature selected from 55°C to 65°C and the solution is cooled at a predefined temperature selected from 0°C to 10°C

In this embodiment, the pure Enzalutamide has about 99.7% to 99.95% HPLC purity with about 50 % to 60 % yield.

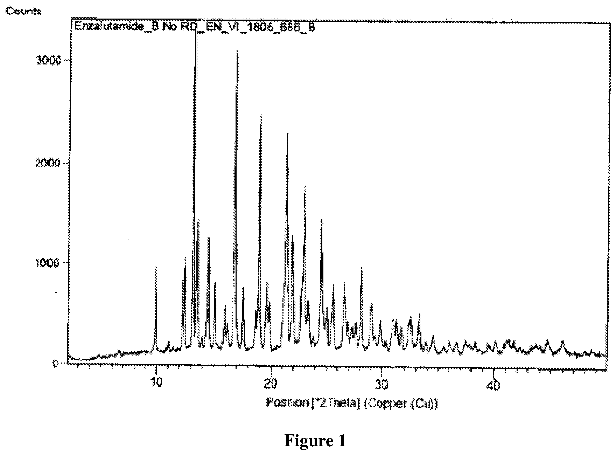

In yet another embodiment, the present invention provides a process for preparation of a crystalline Form 1 of Enzalutamide characterized by a PXRD pattern substantially as illustrated by Figure 1. The process comprises the steps of:
a) preparing solution of pure Enzalutamide in the mixture of predefined solvent; and
b) isolating the crystalline Form 1 of Enzalutamide.

In this embodiment, the mixture of solvents used in step a) are selected from methylene dichloride (MDC), toluene, acetone and methanol. The ratio of Toluene:MDC is 1 - 3 v/v; ratio of Acetone:MDC is 50 - 70 v/v and the ratio of MDC:Methanol is 11 - 15 v/v.

The predefined volume/volume percent of methanol used in step a) ranges from 0.1 v/v to 0.3 v/v, preferably 0.3 v/v.

In one embodiment, isolating crystalline Form 1 of Enzalutamide in step b) by crash cooling or sudden cooling at a temperature ranging from room temperature to -25°C to -30°C. Crystalline Form 1 of Enzalutamide obtained is having purity of 99.7% to 99.95% HPLC purity with yield of 90 % to 99 % yield (on wt/wt basis).

In another embodiment, isolating crystalline Form 1 of Enzalutamide in step b) by adding the solution formed in step a) to a predefined chilled or pre-cooled anti-solvent selected from n-heptane and water. Crystalline Form 1 of Enzalutamide precipitated out is having purity of 99.7% to 99.95% HPLC purity with yield of 90 % to 99 % yield (on wt/wt basis). Addition of solution from step a) to pre-cooled n-heptane is carried at a temperature from -25°C to - 30°C. Addition of solution from step a) to pre-cooled water is carried at a temperature from 0°C to 5°C. The predefined volume of anti-solvent n-heptane used in step b) ranges from 15V to 50 V and predefined volume of water ranges from 15V to 50V.

In yet another embodiment, isolating crystalline Form 1 of Enzalutamide in step b) by evaporating the solvent without vacuum at predefined temperature from 40°C to 55°C to obtain oily mass. Further to the oily mass obtained after evaporation, applying vacuum in the range of 600-750 mm Hg to yield crystalline Form 1 of Enzalutamide having purity of 99.7% to 99.95% HPLC purity with yield of 90 % to 99 % yield (on wt/wt basis).

In this embodiment, Enzalutamide crystalline Form I obtained by the process of the present invention is characterized by X-ray powder diffraction pattern (XRD) and thermal techniques such as melting point and differential scanning calorimetry (DSC) analysis as illustrated in Figure 1. As illustrated from the figure 1 the crystalline Form 1 of Enzalutamide characterized by XRD patterns is showing one or more peaks at 12.39, 13.21, 15.07, 17.53 degrees 2-theta. Further the XRD pattern of Enzalutamide crystalline Form 1 are having one or more additional peaks at 9.90, 13.58, 14.42, 16.79, 18.94, 21.26, 21.91, 22.88, 24.48 degrees 2-theta.

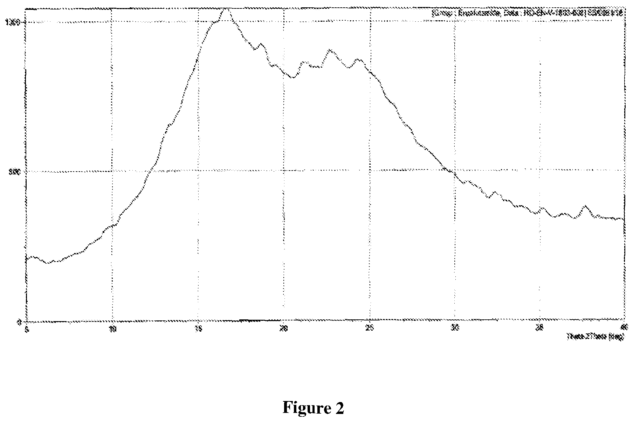

In yet another embodiment, the present invention provides a process for preparation of a amorphous form of Enzalutamide characterized by a PXRD pattern substantially as illustrated by Figure 2. The process comprises the steps of:
A) preparing solution of pure Enzalutamide in a predefined volume of a predefined mixture of solvent; and
B) isolating amorphous form of Enzalutamide.

In this embodiment, the predefined solvents in step A) are selected from methylene dichloride and methanol. The ratio of the solvent mixture is the ratio of the solvent is 7 to 9. The predefined volume/volume percent of methanol used in step A) is 0.5v/v to 2v/v. The methylene dichloride layer in step B) is evaporated at temperature ranging from 50°C to 55°C. Sudden precipitation of Amorphous Form of Enzalutamide is observed to yield Amorphous Form of Enzalutamide having purity of 99.7% to 99.95% HPLC purity with yield of 90 % to 99 % yield (on wt/wt basis).

In this embodiment, Amorphous Form of Enzalutamide obtained by the process of the present invention is characterized by X-ray powder diffraction pattern (XRD) and thermal techniques such as melting point and differential scanning calorimetry (DSC) analysis is illustrated in Figure 2. As illustrated from the figure 2 the amorphous form of Enzalutamide characterized by XRD patterns is having one or more peaks at about 6.5±0.2, 15.86±0.2 and 17.34±0.2 degrees 2-theta.

### EXAMPLES

Only a few examples and implementations are disclosed. Variations, modifications, and enhancements to the described examples and implementations and other implementations can be made based on what is disclosed.

Examples are set forth herein below and are illustrative of different amounts and types of reactants and reaction conditions that can be utilized in practicing the disclosure. It will be apparent, however, that the disclosure can be practiced with other amounts and types of reactants and reaction conditions than those used in the examples, and the resulting devices various different properties and uses in accordance with the disclosure above and as pointed out hereinafter.

### Example 1

### Preparation of 4-Bromo-2-fluoro-N-methylbenzamide (Formula D)

Dimethyl formamide (1 ml) was charged to the mixture of 4-Bromo-2-fluoro benzoic acid (100 g) and toluene (200 ml) with stirring at a temperature from about 30-35°C. The temperature of the reaction mixture was raised to 65-70°C. Thionyl chloride (100 g) was added slowly to the mass at 65-70°C. The mass was stirred for 1-2 hours to get clear solution. The mass was cooled to 10-15°C. The mass was slowly added to the 40% solution of monomethylamine (281 ml) previously cooled to 15°C. The mixture was stirred for 1 hour at 10-15°C. The mass was filtered under vacuum at 50-60 torr. The reaction mass was washed with Toluene (50 ml) followed by washing with water (100 ml). The product was dried in hot air oven at a temperature from about 60 °C to about 65 °C to get dry weight (100 g) having a purity of 99.44%.

### Example 2

### Preparation of 2-[3-fluoro-4-(methylcarbamoyl)anilino]-2-methyl-propanoic acid (Formula III)

Copper chloride (12.8 gm) was charged to N,N'-dimethyl Formamide (600 ml) under argon atmosphere at 75-80°C. 4-Bromo-2-fluoro-N-methylbenzamide obtained in example 1 (100 g) was charged to the mixture followed by addition of N,N-dimethylaniline (8.35 gm). The reaction mixture was stirred for 15-20 minutes. 2-aminoisobutyric acid (67 gm) was added and again the mixture was stirred for 15-20 minutes at 75-80°C. Powdered potassium carbonate (148 gm) was added and stirred for 15 minutes. Water (10 ml) was added and the temperature of the mass was raised to 110°C and the mass was stirred for 4-5 hours at 4-5 hours. The mass was cooled to 30-35°C and filtered. The mass was filtered and washed with dimethyl formamide. Dimethyl formamide was distilled out under vacuum and the mass was cooled. Water (700 ml) was added to the mass and pH was adjusted to 13.5 - 14 by using 10% sodium hydroxide solution. MDC (300 ml) was added to the reaction mass and stirred. The layers were separated and aqueous layer was extracted with MDC (300 ml). MDC layer was separated and washed with water (300 ml). All the aqueous layers were combined and pH was adjusted to 2.5-3 by HCl to precipitate out the product. The mass was stirred and filtered under vacuum. The precipitate was washed with water (100 ml) and suck dried well. The material was dried under vacuum at 55-60°C.

The dried product 2-[3-fluoro-4-(methylcarbamoyl) anilino]-2-methyl-propanoic acid (135 g) was isolated. Thereafter, the product is tested for HPLC purity which was observed to be 99.4%.

### Example 3

### Preparation of methyl N-[4-cyano-3-(trifluoromethyl)phenyl]carbamodithioate through [4-cyano-3-(trifluoromethyl)phenyl]carbamodithioic acid, 1,8-BDU complex

### I) Preparation of [4-cyano-3-(trifluoromethyl)phenyl]carbamodithioic acid, 1,8-BDU complex (Formula V)

4-amino-2-(trifluromethyl benzonitrile (100 g) was added to acetone (200 ml) at 25-30°C. The reaction mixture was cooled to 15-20°C. 1,8-DBU (245 g) was added slowly to the reaction mass at a temperature from about 15 °C to about 20°C and carbon disulfide (211 gm) was added drop wise within 30 minutes at a temperature from about 15 °C to about 20°C. The reaction mass was maintained for 3-4 hours at 25-30°C. The product was precipitated out and the mixture was stirred for 1 hour after precipitation. The reaction mass was quenched in DM water (3 L). The mass was maintained for 30 minutes at a temperature from about 25 °C to about 30°C. The reaction mass was filtered under vacuum and wet cake washed with DM water (100 ml). The cake was suck dried and further dried under vacuum at 40°C to isolate [4-cyano-3-(trifluoromethyl)phenyl]carbamodithioic acid as 1,8-DBU complex (210 g). Thereafter, the product is tested for HPLC purity and LCMS purity such that HPLC purity was observed to be 57.84% and LCMS purity was observed to be 60.15%, [M+1] = 415.

### II) Preparation of methyl N-[4-cyano-3-(trifluoromethyl)phenyl]carbamodithioate (Formula II)

1,8-DBU complex of [4-cyano-3-(trifluoromethyl)phenyl]carbamodithioic acid (100 g) as prepared in step (I) above was charged to DMF (200 ml) at a temperature from about 25 °C to about 30°C. The reaction mixture was cooled to 10-15°C. Methyl iodide (48.58 g) was added drop wise to the reaction mixture. The mass was stirred for 30 minutes at a temperature from about 15°C to about 20°C. Water (3 L) was added to the reaction mass and stirred for 20-30 minutes. The product methyl N-[4-cyano-3-(trifluoromethyl)phenyl] carbamodithioate was separated by filtration under vacuum and washed with DM water (100 ml). The product was suck dried and further dried under vacuum at a temperature of 50°C to about 55°C to isolate dry weight (62 gm). Thereafter, the product is tested for HPLC purity which was observed to be 80%.

### Example 4

### Preparation of methyl N-[4-cyano-3-(trifluoromethyl)phenyl]carbamodithioate through [4-cyano-3-(trifluoromethyl)phenyl]carbamodithioic acid, acetamidine complex

### i) Preparation of [4-cyano-3-(trifluoromethyl)phenyl]carbamodithioic acid, acetamidine complex

4-amino-2-(trifluromethyl benzonitrile (20 g) was charged to acetone (40 ml) at 25-30°C. The reaction mixture was stirred well at 30°C. Acetamidine Hydrochloride (31 g) and Triethylamine (32.64 g) was added to the mixture. The mixture was cooled to 20°C and carbon disulfide (43 g) was charged drop wise till half an hour. The mass was stirred for 8-9 hours. The precipitate obtained was filtered and washed with acetone and suck dried. [4-cyano-3-(trifluoromethyl)phenyl]carbamodithioic acid, acetamidine complex (30 gm) was isolated.

### ii) Preparation of methyl N-[4-cyano-3-(trifluoromethyl)phenyl]carbamodithioate (Formula II)

[4-cyano-3-(trifluoromethyl)phenyl]carbamodithioic acid, acetamidine complex (30 gm) was charged in DMF (60 ml) at room temperature. The reaction mixture was cooled to 10°C and methyl iodide (17.3 g) was added drop wise to the mixture. The mixture was maintained for 1-2 hours. The mass was quenched into water (750 ml) at room temperature. The mass was maintained under continuous stirring for 1-2 hours and MDC (100 ml) was charged. The mass was stirred for 0.5 hours and layers were separated. The aqueous layer was charged to MDC (100 ml) and stirred for 15 minutes. The layers were separated and MDC layer was collectively dried over sodium sulfate. Methyl N-[4-cyano-3-(trifluoromethyl)phenyl]carbamodithioate was isolated.

### Example 5 (comparative)

### Purification of methyl N-[4-cyano-3-(trifluoromethyl)phenyl]carbamodithioate (Formula II)

2-[3-fluoro-4-(methylcarbamoyl) anilino]-2-methyl-propanoic acid (100 g) was added to the mixture of Toluene (390 ml) and methanol (10 ml) at a temperature from about 25 °C to about 30°C to form a slurry. The temperature of reaction mass was raised to 70-75°C till reaction mass becomes clear. The mass was filtered and washed with Toluene (25 ml). The reaction mass was cooled at 10-15°C and stirred for 1 hour. The mass was filtered and washed with cool toluene (25 ml). The precipitate was suck dried and further dried under vacuum at 55-60°C for 6-7 hours. After cooling below 35°C, pure methyl N-[4-cyano-3-(trifluoromethyl)phenyl]carbamodithioate (61 gm) was obtained.

HPLC Purity: 95.05%

### Example 6

### Preparation of Enzalutamide (Formula I)

2-[3-fluoro-4-(methylcarbamoyl) anilino]-2-methyl-propanoic acid (100 g) was added to Toluene (500 ml) at a temperature from about 30 °C to about 35°C. Di-isopropyl ethylamine (101.6 g) was added to the reaction mixture. methyl N-[4-cyano-3-(trifluoromethyl)phenyl] carbamodithioate (163 g) was charged to the reaction mass followed by addition of phenol (111 g) at 30-35°C. The reaction mass was heated to 108-112°C and water was distilled azeotropically. After completion of the reaction, the mass was cooled to a temperature from about 55 °C to about 60°C. Toluene was distilled under vacuum. The mass was cooled to 30-35°C. MDC (600 ml) was charged to the reaction mass and stirred for 30 to 40 minutes. 10% sodium hydroxide solution was charged to the mass and stirred for 30-35 minutes. The mass was filtered and 10% sodium hydroxide (200 ml) was added to the organic layer. The organic layer was separated and 10% sodium hydroxide (100 ml) was charged and stirred for 15-20 minutes. The layers were separated and 10% HCl (300 ml) was added and stirred for 15-20 minutes. The layers were separated and water (250 ml) was charged to the organic layer and stirred for 15-20 minutes. The MDC layer was distilled out at 55 to 60°C. IPA (100 ml) was charged and was distilled under vacuum at 55-60°C. The oily mass was cooled to 30-35°C and IPA (500 ml) was charged. The solid mass was stirred for 60-70 minutes at 30-35°C. The solid mass was filtered and washed with IPA. The precipitate was suck dried and further dried under vacuum at 50-60°C for 6-7 hours. The material was cooled and Enzalutamide (125 g) was unloaded.

HPLC Purity: 97.11%

### Example 7 (comparative)

### Preparation of (2-fluorophenyl) N-[4-cyano-3-(trifluoromethyl)phenyl] carbamodithioate

4-isothiocyanato-2-(trifluoromethyl)benzonitrile (3.3 g) was charged to the mixture of methylene dichloride (10 ml) and 2-fluorothiophene (2 g) and the reaction mixture was stirred at 28°C for 2 hours. Ethyl acetate (100 ml) and water (50 ml) was charged to the reaction mass and stirred for 15 minutes. The organic layer was separated and dried over sodium sulfate. The layer was filtered and concentrated under vacuum to obtain oily mass. The mass was kept for 12 hours at room temperature to yield (2-fluorophenyl) N-[4-cyano-3-(trifluoromethyl)phenyl] carbamodithioate as a yellow powder (3.9 gm).

### Example 8 (comparative)

### Preparation of Enzalutamide (Formula I)

(2-fluorophenyl) N-[4-cyano-3-(trifluoromethyl)phenyl] carbamodithioate (3.9 gm) was charged Toluene (10 ml) cooled at 28°C. Di-isopropyl ethylamine (2.03 gm) and 2-[3-fluoro-4-(methylcarbamoyl) anilino]-2-methyl-propanoic acid (2 gm) was charged in the reaction mixture at 28°C. Phenol (2.2 gm) was charged at 28°C. The temperature was raised to 110°C and maintained for 4 hours. The reaction mass was gradually cooled to room temperature and further to 10-15°C. Methylene dichloride (50 ml) was added to the reaction mass. 5% NaOH solution (25 ml) was added and mass was stirred well for 10 minutes. The organic layer was separated and washed with 5% NaOH (25 ml). The organic layer was stripped for 25 minutes with 10% hydrochloric acid. The layers were separated and washed with water and dried over sodium sulfate. The organic layer was concentrated on rotavapor at 55-60°C. IPA (5 ml) was charged and slurry obtained was stirred for 30 minutes. The slurry was filtered and dried well to yield Enzalutamide (1.8 gm).

### Example 9 (comparative)

### Preparation of (4-fluorophenyl) N-[4-cyano-3-(trifluoromethyl)phenyl] carbamodithioate

4-isothiocyanato-2-(trifluoromethyl)benzonitrile (8.8 g) was charged to methylene dichloride (25 ml) followed by addition of 4-fluorothiophene (4.1 g) and the reaction mixture was stirred at 25°C for 7 hours. Methylene dichloride (25 ml) was charged to the reaction mass and stirred for 10 minutes. 10% NaOH solution (25 ml) was added and stirred for 5 minutes. The organic layer was separated and dried over sodium sulfate and filtered and concentrated under vacuum at 50-55°C for 1 hour to yield (4-fluorophenyl) N-[4-cyano-3-(trifluoromethyl)phenyl] carbamodithioate as yellow powder (12 gm).

he organic layer was separated and dried over sodium sulfate. The layer was filtered and concentrated under vacuum to obtain oily mass. The mass was kept for 12 hours at room temperature to yield (4-fluorophenyl) N-[4-cyano-3-(trifluoromethyl)phenyl] carbamodithioate as a yellow powder (3.9 gm).

### Example 10 (comparative)

### Preparation of Enzalutamide (Formula I)

2-[3-fluoro-4-(methylcarbamoyl) anilino]-2-methyl-propanoic acid (3.9 gm) was charged to Toluene (15 ml) which is preciously cooled to 28°C. Di-isopropyl ethylamine (2.03 gm) and (4-fluorophenyl) N-[4-cyano-3-(trifluoromethyl)phenyl] carbamodithioate (5.8 gm) was charged in the reaction mixture at 28°C. Phenol (3.3 gm) was charged at 28°C. The temperature was raised to 110°C and maintained for 2 hours.

The reaction mass was gradually cooled to room temperature. Toluene was distilled out and Methylene dichloride (30 ml) was added to the reaction mass. 10% NaOH solution (30 ml) was added and mass was stirred well for 30 minutes. The organic layer was separated and 10% HCl (30 ml) was charged and stirred for 15 minutes . The layers were separated and washed with water and dried over sodium sulfate. The organic layer was concentrated on rotavapor and degassed at 55-60°C under vacuum. IPA (15 ml) was charged and slurry obtained was stirred for 30 minutes. The slurry was filtered and dried well to yield Enzalutamide (3.1 gm).

### Example 11 (comparative)

### Phenyl N-[4-cyano-3-(trifluoromethyl)phenyl] carbamodithioate (Formula II)

2-isothiocyanato-2-(trifluoromethyl)benzonitrile (3.55 g) and MDC (10 ml) was charged to Thiophenol (1.7 gm) at 25-30°C. The reaction mixture was stirred at room temperature for 3 hours. Ethyl acetate (100 ml) and water (100 ml) was charged to the reaction mixture at room temperature. The organic layer was separated and organic layer was dried over sodium sulfate. The layer was concentrate under vacuum at 50-55°C to get Phenyl N-[4-cyano-3-(trifluoromethyl)phenyl] carbamodithioate (4.6 gm).

### Example 12 (comparative)

### Preparation of Enzalutamide (Formula I)

2-[3-fluoro-4-(methylcarbamoyl) anilino]-2-methyl-propanoic acid (3.9 gm) was charged to Toluene (15 ml) which is previously cooled to 28°C. Di-isopropyl ethylamine (2.33 gm) and fluorophenyl-N-[4-cyano-3-(trifluoromethyl)phenyl] carbamodithioate (5.1 gm) was charged in the reaction mixture at 28°C. Phenol (2.77 gm) was charged at 28°C. The temperature was raised to 110°C and maintained for 3.5 hours.

The reaction mass was gradually cooled to room temperature. Toluene was distilled out and Methylene dichloride (25 ml) was added to the reaction mass. 5% NaOH solution (25 ml) was added and mass was stirred well for 10 minutes. The organic layer was separated and 5% NaOH solution (25 ml) was added to organic layer and stirred for 10 minutes. The organic layer was separated and 10% HCl (25 ml) was charged to the toluene layer and stirred for 10 minutes. The organic layer was separated and concentrated under vacuum at 50-55°C. The oily mass was slurried with IPA (5 ml) and filtered and suck dried.

The precipitate was dried at 50°C to isolate Enzalutamide (2 gm).

### Example 13 - Comparative Example (For Dimer impurity)

### Synthesis of Enzalutamide

4-[[2-(dimethylamino)-1,1-dimethyl-2-oxo-ethyl]amino]-2-fluoro-N-methyl-benzamide (25 gm) was added to DMSO (25 ml). Ethyl acetate (55 ml) was charged to the reaction mixture. 4-[[2-(dimethylamino)-1,1-dimethyl-2-oxo-ethyl]amino]- 2-fluoro-N-methyl-benzamide (45.5 gm) was added to the mixture. The mixture was heated to gradually heated to 80°C. The reaction was maintained for 20-22 hours at 80°C. After completion of reaction the mass was sent for analysis.

Analogous impurities was found to be 21.18% and dimer impurity was 1.67%
Water (250 ml) was charged to the reaction mass. The mass was extracted twice with MDC (100 ml x2). The organic layer was separated and dried over sodium sulfate. MDC layer was filtered and distilled out under vacuum. IPA stripping (100 ml x2) was given to the oily mass obtained. The solid obtained was filtered and washed with IPA. Crude Enzalutamide (28 gm wet wt) was isolated, which on drying yields 27 gm Enzalutamide (yield: 62.3%).

Analogous impurities was found to be 15.80% and dimer impurity was 0.15%

### Example 14

### Purification of Enzalutamide

Crude Enzalutamide (100 gm) obtained from any of the above examples was charged to methanol (1000 ml) at room temperature. The temperature of the mixture was raised to 55-65°C. Activated charcoal (5 gm) was added to the clear solution, filtered through hyflo and washed with methanol at 55-65°C. The mass was cooled to 0 to 10°C and stirred for 1 hour. The mass was filtered and washed with cool methanol. Pure Enzalutamide (55 gm) was isolated after drying.

### Example 15

### Preparation of Enzalutamide polymorphic Form 1

Pure Enzalutamide (100 gm) obtained in example 10, was charged to acetone (150 ml). MDC (2.5 ml) was added to the reaction mixture at 25-30°C. The temperature was raised to 35-40°C. The mixture was slowly added to previously chilled n-heptane to -25 to -30°C (2500 ml). The mass was stirred at -25 to -30°C for 1 hour. The temperature of the mass was slowly raised to 25-30°C and stirred at 25-30°C for 2 hours. The mass was filtered and washed with n-heptane. Crystalline Enzalutamide obtained was dried under vacuum at 50-60°C for 6 hours. It was cooled to room temperature to give Polymorphic Form 1 (90 gm).
Yield: 96 gm
HPLC purity: 99.90%

### Example 16

### Preparation of Enzalutamide polymorphic Form 1

Pure Enzalutamide (10 gm) obtained in example 10, was charged to acetone (150 ml). MDC (2.5 ml) was added to the reaction mixture at 25-30°C. The temperature was raised to 35-40°C. The mixture was slowly added to previously cooled DM water to 0 to 5°C (250 ml). The mass was stirred at 0 to 5°C for 2 hour. The mass was filtered and washed with DM water. Crystalline Enzalutamide obtained was dried under vacuum at 50-60°C for 6 hours. It was cooled to room temperature to give Polymorphic Form 1 (90 gm).

Yield: 4gm

The Powder X-ray diffraction (PXRD) pattern of Enzalutamide polymorphic Form 1 obtained in above example 15 is in accordance with Figure 1.

### Example 17

### Preparation of Enzalutamide polymorphic Form 1

Enzalutamide (50 gm) was charged to MDC (200 ml) followed by addition of methanol (15 ml). The solvent was distilled out without vacuum at 51°C to yield oily mass. Vacuum was applied till solid appears. The compound was dried at 50-55°C under vacuum to yield Form 1 (49 gm). (Yield 98%).

The Powder X-ray diffraction (PXRD) pattern of Enzalutamide polymorphic Form 1 obtained in above example 16 is in accordance with Figure 1.

### Example 18

### Preparation of Enzalutamide polymorphic Form 1

Enzalutamide (100 gm) was charged to MDC (200 ml) and stirred to get clear solution.

Toluene (300 ml) was added and the temperature was raised to 60-65°C. MDC was distilled out atmospherically at 60-65°C. The mass was cooled to 20-25°C, stirred and filtered. The precipitate was washed with toluene (50 ml) at 25-30°C and suck dried and dried under vacuum at 55-60°C for 6-7 hours. Enzalutamide Form 1 obtained was isolated (95 gm).

The Powder X-ray diffraction (PXRD) pattern of Enzalutamide polymorphic Form 1 obtained in above example 17 is in accordance with Figure 1.

### Example 19 (comparative)

### Preparation of Enzalutamide amorphous Form

Enzalutamide (5 gm) was charged to MDC (20 ml) at room temperature. Methanol (2.5 ml) was charged to the mixture at room temperature. MDC layer was evaporated at 50-55°C without vacuum. Vacuum was applied to the oily liquid obtained at 51°C. Fluffy solid obtained was dried at 50-55°C under vacuum to yield 4.7 gm amorphous Enzalutamide (Yield: 94%).

The Powder X-ray diffraction (PXRD) pattern of Enzalutamide amorphous Form obtained in above example 18 is in accordance with Figure 2.

In the context of the present invention, the process of the present invention is an eco-friendly and a cost effective process. The process of the present invention advantageously avoids use of hazardous reagents like triphosgene, thiophosgene and acetone cyanohydrin. The process of the present invention results in high yield of the end product of the present invention with maximum HPLC purity. The process present invention are reproducible and are scalable at higher scale, which consistently provides pure crystalline Form I and amorphous form of Enzalutamide with high purity.

The foregoing description of specific embodiments of the present invention has been presented for purposes of illustration and description. They are not intended to be exhaustive or to limit the present invention to the precise forms disclosed, and obviously many modifications and variations are possible in light of the above teaching.

The embodiments were chosen and described in order to best explain the principles of the present invention and its practical application, to thereby enable others, skilled in the art to best utilize the present invention and various embodiments with various modifications as are suited to the particular use contemplated.

It is understood that various omission and substitutions of equivalents are contemplated as circumstance may suggest or render expedient, but such are intended to cover the application or implementation without departing from the scope of the present invention, which is defined by the claims.

## Claims

1. A process for preparation of Enzalutamide of Formula (I), comprising: 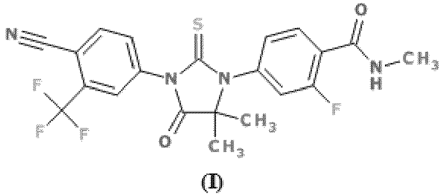 reacting a compound of formula (II) 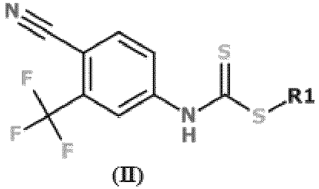
wherein R1 is (1-4C) alkyl, (1-4C) alkyl substituted by ethyl acetate or benzyl acetate, benzyl or benzyl substituted at ortho or para-positions by halo, NO₂ or CN;
with a compound of formula (III) 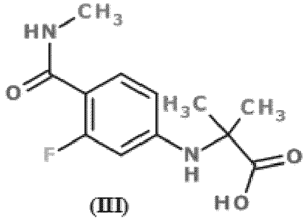
at a temperature of from 60°C to 140°C in presence of a tertiary amine base in a solvent to form Enzalutamide of Formula (I).

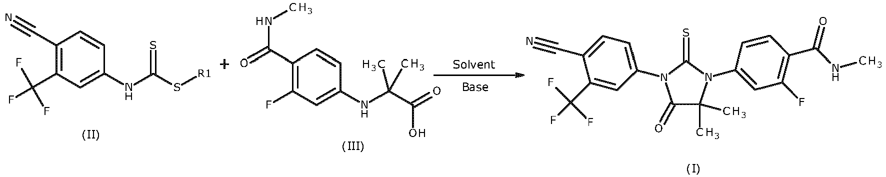

2. The process as claimed in claim 1, wherein the tertiary amine base is selected from the group consisting of di-isopropyl ethylamine (DIPEA), triethylamine, trimethylamine, pyridine; preferably di-isopropylethylamine.

3. The process as claimed in claim 1, wherein the solvent is selected from toluene, xylene, cyclohexane, chlorobenzene, dichlorobenzene; and/or wherein the predefined tertiary amine base to solvent ratio is 0.15 to 0.25 (wt/v).

4. The process as claimed in claim 1, wherein the process of preparation of compound of formula (II) comprising;
1) reacting compound of formula (A) 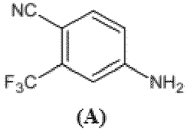 with carbondisulfide at a temperature of from 15°C to 20°C in presence of amidine base or a 1,4-diazabicyclo[2.2.2]octane (DABCO) or a DABCO derivative and a solvent at room temperature to form a compound of formula (IIA), wherein the solvent used is a ketonic solvent selected from acetone, methyl ethyl ketone, or methyl isobutyl ketone; and 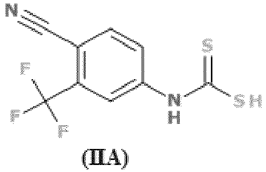
2) reacting the compound of formula (IIA) with an alkylating agent in presence of a solvent at room temperature to form compound of formula (II), wherein the solvent used is a ketonic solvent, a polar aprotic solvent or an ethereal solvent.

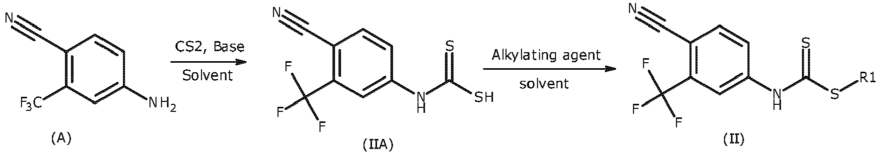

5. The process as claimed in claim 4, wherein the amidine base is selected from the group consisting of acetamidine, benzamidine, diazabycyclo[5.4.0]undec-7-ene (DBU), diazabycyclo[4.3.0]non-5-ene (DBN) and imidazolidine.

6. The process as claimed in claim 4, wherein the solvent used in step 1) is acetone.

7. The process as claimed in claim 4, wherein the compound of formula (II) formed in step 2) is isolated as an amidine base complex or (1,4-diazabicyclo[2.2.2]octane) DABCO salt as compound of formula (IV).

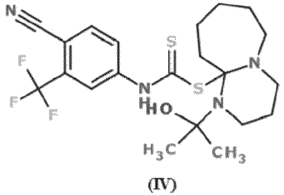

8. The process as claimed in claim 4, wherein the alkylating agent is selected from (1-4 C) alkyl halide, benzyl halide, o-nitro benzyl halide, p-nitrobenzyl halide, o-cyano benzyl halide, p-cyano benzyl halide chloro ethylacetate, bromo ethylacetate, iodo ethylacetate, chloro benzylacetate, bromo benzylacetate and iodo benzylacetate.

9. The compound of formula (II) as claimed in claim 1 and 4, wherein the compound of formula (II) is an alkyl or benzyl ester of [4-cyano-3-(trifluoromethyl)phenyl]carbamodithioic acid); 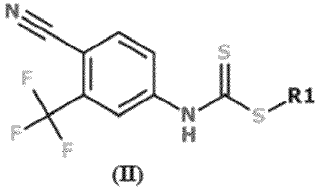 wherein R1 is (1-4C) alkyl, (1-4C) alkyl substituted by ethyl acetate or benzyl acetate, benzyl or benzyl substituted at ortho or para-positions by halo, NO2 or CN.

10. The process of claim 1 further comprising the steps of:
a) preparing a solution of Enzalutamide in a mixture of solvent selected from methylene dichloride (MDC), acetone, toluene and methanol; optionally wherein the ratio of Toluene to MDC is 1 to 3 v/v, the ratio of Acetone to MDC is 50 to 70 v/v; and the ratio of MDC to Methanol is 11 to 15 v/v; and
b) isolating a crystalline Form 1 of Enzalutamide by either:
i. adding the solution formed in step a) to a mixture of chilled or a pre-cooled solvent, optionally n-heptane at -25 to -30 °C; or
ii. by evaporating the solvent without vacuum and applying a predefined vacuum; optionally wherein the predefined vacuum is in the range of 600-750 mm Hg.

## Patentansprüche

1. Verfahren zur Herstellung von Enzalutamid der Formel (I), umfassend: 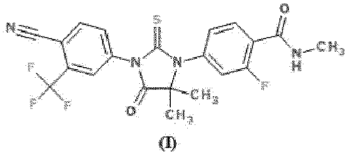 Umsetzen einer Verbindung der Formel (II) 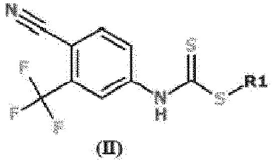
wobei R1 (1-4C)-Alkyl, (1-4C)-Alkyl, das mit Ethylacetat oder Benzylacetat substituiert ist, Benzyl oder Benzyl ist, das an der Ortho- oder para-Position durch Halogen, NO₂ oder CN substituiert ist;
mit einer Verbindung der Formel (III) 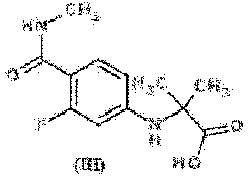
bei einer Temperatur von 60 °C bis 140 °C in Gegenwart einer tertiären Aminbase in einem Lösungsmittel, um Enzalutamid der Formel (I) zu bilden

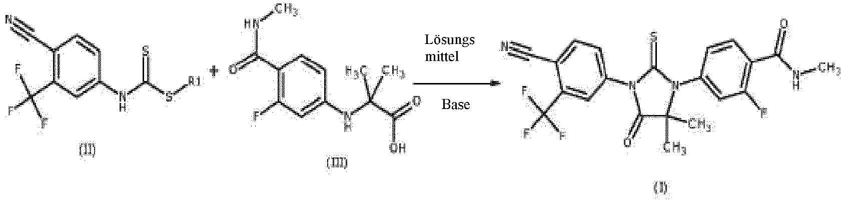

2. Verfahren nach Anspruch 1, wobei die tertiäre Aminbase ausgewählt ist aus der Gruppe bestehend aus Diisopropylethylamin (DIPEA), Triethylamin, Trimethylamin, Pyridin; vorzugsweise Diisopropylethylamin.

3. Verfahren nach Anspruch 1, wobei das Lösungsmittel ausgewählt ist aus Toluol, Xylol, Cyclohexan, Chlorbenzol, Dichlorbenzol; und/oder wobei das vordefinierte Verhältnis von tertiärer Aminbase zu Lösungsmittel 0,15 bis 0,25 (Gew./Vol.) beträgt.

4. Verfahren nach Anspruch 1, wobei das Verfahren der Herstellung der Verbindung der Formel (II) umfasst:
1) Umsetzen der Verbindung der Formel (A) 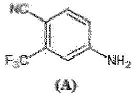 mit Kohlenstoffdisulfid bei einer Temperatur von 15 °C bis 20 °C in Gegenwart von Amidinbase oder einem 1,4-Diazabicyclo[2.2.2]octan (DABCO) oder einem DABCO-Derivat und einem Lösungsmittel bei Raumtemperatur, um eine Verbindung der Formel (IIA) zu bilden, wobei das verwendete Lösungsmittel ein ketonisches Lösungsmittel ausgewählt aus Aceton, Methylethylketon oder Methylisobutylketon ist; und 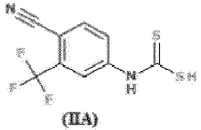
2) Umsetzen der Verbindung der Formel (IIA) mit einem Alkylierungsmittel in Gegenwart eines Lösungsmittels bei Raumtemperatur, um die Verbindung der Formel (II) zu bilden, wobei das verwendete Lösungsmittel ein ketonisches Lösungsmittel, ein polar-aprotisches Lösungsmittel oder ein etherisches Lösungsmittel ist

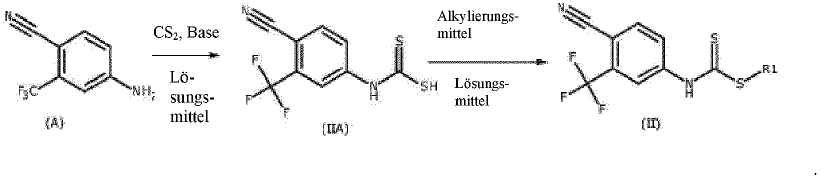

5. Verfahren nach Anspruch 4, wobei die Amidinbase ausgewählt ist aus der Gruppe bestehend aus Acetamidin, Benzamidin, Diazabicyclo[5.4.0]undec-7-en (DBU), Diazabicyclo[4.3.0]non-5-en (DBN) und Imidazolidin.

6. Verfahren nach Anspruch 4, wobei das in Schritt 1) verwendete Lösungsmittel Aceton ist.

7. Verfahren nach Anspruch 4, wobei die Verbindung der Formel (II), die in Schritt 2) gebildet wurde, als Amidinbasenkomplex oder (1,4-Diazabicyclo[2.2.2]octan) (DABCO)-Salz als Verbindung der Formel (IV) isoliert wird

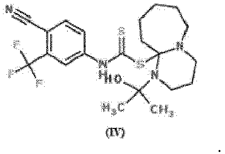

8. Verfahren nach Anspruch 4, wobei das Alkylierungsmittel ausgewählt ist aus (1-4C)-Alkylhalogenid, Benzylhalogenid, o-Nitrobenzylhalogenid, p-Nitrobenzylhalogenid, o-Cyanobenzylhalogenid, p-Cyanobenzylhalogenid, Chlorethylacetat, Bromethylacetat, Iodethylacetat, Chlorbenzylacetat, Brombenzylacetat und Iodbenzylacetat.

9. Verbindung der Formel (II) nach Anspruch 1 und 4, wobei die Verbindung der Formel (II) ein Alkyl- oder Benzylester von [4-Cyano-3-(trifluormethyl)phenyl]-carbamodithiosäure) ist 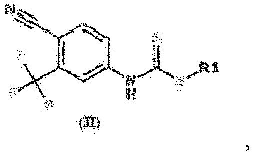 wobei R1 (1-4C)-Alkyl, (1-4C)-Alkyl, das mit Ethylacetat oder Benzylacetat substituiert ist, Benzyl oder Benzyl ist, das an der ortho- oder para-Position durch Halogen, NO₂ oder CN substituiert ist.

10. Verfahren nach Anspruch 1, des Weiteren umfassend die Schritte:
a) Herstellen einer Lösung von Enzalutamid in einer Mischung von Lösungsmittel ausgewählt aus Dichlormethan (MDC), Aceton, Toluol und Methanol; wobei das Verhältnis von Toluol zu MDC gegebenenfalls 1 bis 3 Vol./Vol. beträgt, das Verhältnis von Aceton zu MDC 50 bis 70 Vol./Vol. beträgt; und das Verhältnis von MDC zu Methanol 11 bis 15 Vol./Vol. beträgt, und
b) Isolieren einer kristallinen Form 1 von Enzalutamid durch entweder:
i. Zufügen der in Schritt a) gebildeten Lösung zu einer Mischung aus gekühltem oder einem vorgekühlten Lösungsmittel, gegebenenfalls n-Heptan, bei -25 bis -30 °C; oder
ii. Verdampfen des Lösungsmittels ohne Vakuum und Anlegen eines vordefinierten Vakuums; wobei das vordefinierte Vakuum gegebenenfalls in einem Bereich von 600 bis 750 mm Hg liegt.

## Revendications

1. Procédé de préparation d'un enzalutamide de la formule (I), comprenant : 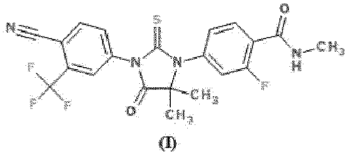 la réaction d'un composé de la formule (II) 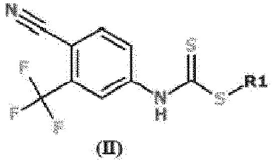
dans lequel R1 est un groupe alkyle en C₁ à C₄, alkyle en C₁ à C₄ substitué par un acétate d'éthyle ou un acétate de benzyle, un groupe benzyle ou benzyle substitué aux positions ortho ou para par un atome halogène, un groupe NO₂ ou CN ;
avec un composé de la formule (III) 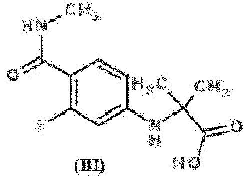
à une température de 60 °C à 140 °C en présence d'une base d'amine tertiaire dans un solvant pour former un enzalutamide de la formule (I).

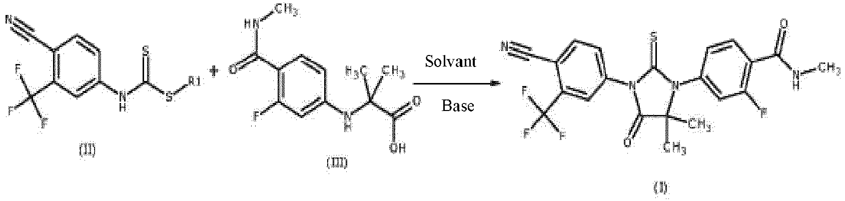

2. Procédé selon la revendication 1, dans lequel la base d'amine tertiaire est choisie parmi le groupe constitué de la di-isopropyléthylamine (DIPEA), de la triéthylamine, de la triméthylamine, de la pyridine ; de préférence la di-isopropyléthylamine.

3. Procédé selon la revendication 1, dans lequel le solvant est choisi parmi le toluène, le xylène, le cyclohexane, le chlorobenzène, le dichlorobenzène ; et/ou dans lequel le ratio prédéfini de la base d'amine tertiaire au solvant est de 0,15 à 0,25 (p/v).

4. Procédé selon la revendication 1, dans lequel le procédé de préparation du composé de la formule (II) comprend :
1) la réaction du composé de la formule (A) 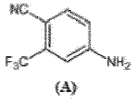 avec du disulfure de carbone à une température de 15 °C à 20 °C en présence d'une base d'amidine, d'un 1,4-diazabicyclo[2.2.2]octane (DABCO) ou d'un dérivé de DABCO et d'un solvant à la température ambiante pour former un composé de la formule (IIA), dans laquelle le solvant utilisé est un solvant cétonique choisi parmi l'acétone, la méthyléthylcétone ou la méthylisobutylcétone ; et 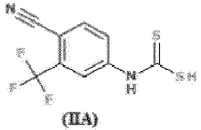
2) la réaction du composé de la formule (IIA) avec un agent alkylant en présence d'un solvant à la température ambiante pour former un composé de la formule (II), dans laquelle le solvant utilisé est un solvant cétonique, un solvant aprotique polaire ou un solvant éthéré.

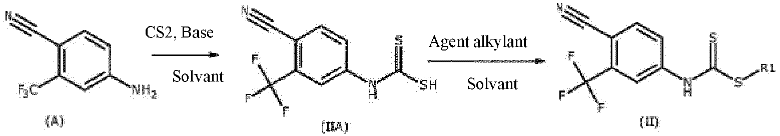

5. Procédé selon la revendication 4, dans lequel la base d'amidine est choisie parmi le groupe constitué de l'acétamidine, la benzamidine, le diazabycyclo[5.4.0]undéc-7-ène (DBU), le diazabycyclo[4.3.0]non-5-ène (DBN) et l'imidazolidine.

6. Procédé selon la revendication 4, dans lequel le solvant utilisé à l'étape 1) est l'acétone.

7. Procédé selon la revendication 4, dans lequel le composé de la formule (II) formé à l'étape 2) est isolé sous forme d'un complexe de base d'amidine ou un sel de (1,4-diazabicyclo[2.2.2]octane) DABCO comme composé de la formule (IV).

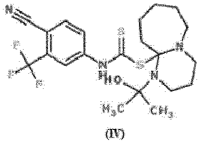

8. Procédé selon la revendication 4, dans lequel l'agent alkylant est choisi parmi un halogénure d'alkyle en C₁ à C₄, un halogénure de benzyle, un halogénure d'o-nitrobenzyle, un halogénure de p-nitrobenzyle, un halogénure d'o-cyanobenzyle, un halogénure de p-cyanobenzyle, un acétate de chloroéthyle, un acétate de bromoéthyle, un acétate d'iodoéthyle, un acétate de chlorobenzyle, un acétate de bromobenzyle et un acétate d'iodobenzyle.

9. Composé de la formule (II) selon les revendications 1 et 4, dans lequel le composé de la formule (II) est un groupe alkyle ou un ester de benzyle de l'acide [4-cyano-3-(trifluorométhyl)phényl]carbamodithioïque ; 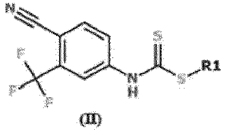 dans lequel R1 est un groupe alkyle en C₁ à C₄, alkyle en C₁ à C₄ substitué par un acétate d'éthyle ou un acétate de benzyle, benzyle ou benzyle substitué aux positions ortho ou para par un atome halogène, un groupe NO₂ ou CN.

10. Procédé selon la revendication 1, comprenant en outre les étapes consistant à :
a) préparer une solution d'enzalutamide dans un mélange de solvants choisis parmi le dichlorure de méthylène (MDC), l'acétone, le toluène et le méthanol ; éventuellement dans lequel le ratio de toluène au MDC est de 1 à 3 v/v, le ratio d'acétone au MDC est de 50 à 70 v/v et le ratio de MDC au méthanol est de 11 à 15 v/v ; et
b) isoler une forme 1 cristalline de l'enzalutamide en :
i. ajoutant la solution formée à l'étape a) à un mélange de solvants refroidis ou prérefroidis, éventuellement du n-heptane à -25 à -30 °C ; ou
ii. évaporant le solvant sans vide et en appliquant un vide prédéfini ; éventuellement dans lequel le vide prédéfini est dans la plage de 600 à 750 mm Hg.
